# EUROPEAN PATENT SPECIFICATION

(11) **EP 4 196 116 B1**
(45) Date of publication and mention of the grant of the patent: **10.07.2024**
(21) Application number: 21766393.9
(22) Date of filing: 12.08.2021
(51) Int. Cl.: A61K 31/395, A61K 31/444, A61K 31/47, A61K 31/517, A61K 45/06, A61P 35/00

(54) **PHARMACEUTICAL COMPOSITION COMPRISING CABOZANTINIB, PLERIXAFOR, AFATINIB AND ETORICOXIB FOR USE IN THE TREATMENT OR PROPHYLAXIS OF NSCLC WITHOUT DRIVER MUTATIONS**
PHARMAZEUTISCHE ZUSAMMENSETZUNG, UMFASSEND CABOZANTINIB, PLERIXAFOR, AFATINIB UND ETORICOXIB ZUR VERWENDUNG BEI DER BEHANDLUNG ODER PROPHYLAXE VON NSCLC OHNE DRIVERMUTATIONEN
COMPOSITION PHARMACEUTIQUE COMPRENANT DU CABOZANTINIB, DU PLERIXAFOR, DE L'AFATINIB ET DE L'ÉTORICOXIB POUR UNE UTILISATION DANS LE TRAITEMENT OU LA PROPHYLAXIE DU CPNPC SANS MUTATIONS ONCOGENIQUES

(30) Priority: 17.08.2020 DE 102020005002
(43) Date of publication of application: 21.06.2023
(73) Proprietor: Langhammer, Stefan, 30938 Burgwedel (DE); EPO Experimentelle Pharmakologie & Onkologie Berlin-Buch GmbH, 13125 Berlin (DE)
(72) Inventor: HOFFMANN, Jens, 13125 Berlin (DE); GÜRGEN, Dennis, 13409 Berlin (DE); LANGHAMMER, STEFAN, Dr., 30938 Burgwedel (DE)
(74) Representative: Prinz & Partner mbB
(86) International application number: PCT/EP2021/072486
(87) International publication number: WO 2022/038038

(56) References cited:
- WO-A1-2012/031027
- WO-A1-2014/039971
- WO-A1-2015/164869
- GRIDELLI C. ET AL: "Selective Cyclooxygenase-2 Inhibitors and Non-small Cell Lung Cancer", CURRENT MEDICINAL CHEMISTRY, vol. 9, no. 21, 1 November 2002 (2002-11-01), NL, pages 1851 - 1858, XP55866070, ISSN: 0929-8673, DOI: 10.2174/0929867023368863
- JUNG M-J ET AL: "Upregulation of CXCR4 is functionally crucial for maintenance of stemness in drug-resistant non-small cell lung cancer cells", ONCOGENE, vol. 32, no. 2, 27 February 2012 (2012-02-27), London, pages 209 - 221, XP55866839, ISSN: 0950-9232, Retrieved from the Internet <URL:http://www.nature.com/articles/onc201237> DOI: 10.1038/onc.2012.37
- LI JIANMIN ET AL: "mir-1-mediated paracrine effect of cancer-associated fibroblasts on lung cancer cell proliferation and chemoresistance", ONCOLOGY REPORTS, vol. 35, no. 6, 29 March 2016 (2016-03-29), pages 3523 - 3531, XP55866062, ISSN: 1021-335X, DOI: 10.3892/or.2016.4714
- NADDA N. ET AL: "Angiostatic role of the selective cyclooxygenase-2 inhibitor etoricoxib (MK0663) in experimental lung cancer", BIOMEDICINE & PHARMACOTHERAPY, vol. 66, no. 6, 1 September 2012 (2012-09-01), FR, pages 474 - 483, XP55866073, ISSN: 0753-3322, DOI: 10.1016/j.biopha.2012.04.002
- NELSON-TAYLOR SARAH ET AL: "Supplementary Figures: Resistance to RET-Inhibition in RET-Rearranged NSCLC Is Mediated By Reactivation of RAS/MAPK Signaling", 1 January 2017 (2017-01-01), XP55866520, Retrieved from the Internet <URL:https://mct.aacrjournals.org/highwire/filestream/62889/field_highwire_adjunct_files/0/176867_2_supp_3997337_mmmmyw.pdf> [retrieved on 20211126], DOI: 10.1158/1535-7163.MCT-17-0008
- NELSON-TAYLOR SARAH K. ET AL: "Resistance to RET-Inhibition in RET-Rearranged NSCLC Is Mediated By Reactivation of RAS/MAPK Signaling", MOLECULAR CANCER THERAPEUTICS, vol. 16, no. 8, 12 May 2017 (2017-05-12), US, pages 1623 - 1633, XP55866037, ISSN: 1535-7163, DOI: 10.1158/1535-7163.MCT-17-0008
- BENJAMIN L LAMPSON ET AL: "Activity of erlotinib when dosed below the maximum tolerated dose for EGFR-mutant lung cancer: Implications for targeted therapy development", CANCER, AMERICAN CANCER SOCIETY , PHILADELPHIA , PA, US, vol. 122, no. 22, 15 August 2016 (2016-08-15), pages 3456 - 3463, XP071137503, ISSN: 0008-543X, DOI: 10.1002/CNCR.30270
- NELSON-TAYLOR SARAH K. ET AL: "Resistance to RET-Inhibition in RET-Rearranged NSCLC Is Mediated By Reactivation of RAS/MAPK Signaling", MOLECULAR CANCER THERAPEUTICS, vol. 16, no. 8, 12 May 2017 (2017-05-12), US, pages 1623 - 1633, XP055866037, ISSN: 1535-7163, Retrieved from the Internet <URL:https://scholar.google.com/scholar_url?url=https://mct.aacrjournals.org/content/molcanther/16/8/1623.full.pdf&hl=de&sa=T&oi=ucasa&ct=ufr&ei=evCgYfaYEpD2yAT97YOQBw&scisig=AAGBfm2muMjFSHJ_V3WQ7qVL69PNFsQv7w> DOI: 10.1158/1535-7163.MCT-17-0008
- NELSON-TAYLOR SARAH ET AL: "Supplementary Figures: Resistance to RET-Inhibition in RET-Rearranged NSCLC Is Mediated By Reactivation of RAS/MAPK Signaling", 1 January 2017 (2017-01-01), XP055866520, Retrieved from the Internet <URL:https://mct.aacrjournals.org/highwire/filestream/62889/field_highwire_adjunct_files/0/176867_2_supp_3997337_mmmmyw.pdf> [retrieved on 20211126], DOI: 10.1158/1535-7163.MCT-17-0008
- JUNG M-J ET AL: "Upregulation of CXCR4 is functionally crucial for maintenance of stemness in drug-resistant non-small cell lung cancer cells", ONCOGENE, vol. 32, no. 2, 27 February 2012 (2012-02-27), London, pages 209 - 221, XP055866839, ISSN: 0950-9232, Retrieved from the Internet <URL:http://www.nature.com/articles/onc201237> DOI: 10.1038/onc.2012.37
- LI JIANMIN ET AL: "mir-1-mediated paracrine effect of cancer-associated fibroblasts on lung cancer cell proliferation and chemoresistance", ONCOLOGY REPORTS, vol. 35, no. 6, 29 March 2016 (2016-03-29), pages 3523 - 3531, XP055866062, ISSN: 1021-335X, Retrieved from the Internet <URL:https://www.spandidos-publications.com/10.3892/or.2016.4714/download> DOI: 10.3892/or.2016.4714
- GRIDELLI C. ET AL: "Selective Cyclooxygenase-2 Inhibitors and Non-small Cell Lung Cancer", CURRENT MEDICINAL CHEMISTRY, vol. 9, no. 21, 1 November 2002 (2002-11-01), NL, pages 1851 - 1858, XP055866070, ISSN: 0929-8673, Retrieved from the Internet <URL:https://www.eurekaselect.com/64213/article> DOI: 10.2174/0929867023368863
- NADDA N. ET AL: "Angiostatic role of the selective cyclooxygenase-2 inhibitor etoricoxib (MK0663) in experimental lung cancer", BIOMEDICINE & PHARMACOTHERAPY, vol. 66, no. 6, 1 September 2012 (2012-09-01), FR, pages 474 - 483, XP055866073, ISSN: 0753-3322, Retrieved from the Internet <URL:https://www.sciencedirect.com/science/article/pii/S0753332212000509/pdfft?md5=18567ec97505700765f11c3389a71cf3&pid=1-s2.0-S0753332212000509-main.pdf> DOI: 10.1016/j.biopha.2012.04.002
- SANDLER A B ET AL: "COX-2 inhibition and lung cancer", SEMINARS IN ONCOLOGY, W.B. SAUNDERS, US, vol. 31, no. suppl. 7, 1 April 2004 (2004-04-01), pages 45 - 52, XP009138749, ISSN: 0093-7754, DOI: 10.1053/J.SEMINONCOL.2004.03.045

## Description

### FIELD OF INVENTION

The present invention relates to a pharmaceutical composition comprising a plurality of active agents respectively inhibiting VEGF's and/or VEGFR1-3; SDF1alpha and/or CXCR4; EGF and/or EGFR1-3; COX2 and/or PGE2 and/or EP; and HGF and/or HGFR (MET), in particular for use in the treatment or prophylaxis of tumor diseases selected from non-small cell lung cancer (NSCLC).

### BACKGROUND

Drug-based tumor therapy has made immense progress in recent decades. A large number of very differentiated drugs are available for the therapy of tumor diseases.

As an example, Epidermal Growth Factor Receptor (EGFR) - Tyrokinase Inhibitors (TKIs), in particular EGFR1-3 can be used to target proliferation of malignant tumor cells, if present, to their driver mutations, such as EGFR-mutated tumors. Using EGFR1-3 tyrosine kinase inhibitors, such as afatinib, erlotinib, osimertinib, or gefitinib, for the first-line treatment of advanced NSCLC with activating EGFR mutations is well established. However, treatment options after development of resistance for advanced squamous NSCLC, are limited, particularly in the second-line setting after progression. Vascular Endothelial Growth Factor (VEGF) inhibitors or Vascular Endothelial Growth Factor Receptors (VEGFR) inhibitors can be used to target tumor endothelial cells in order to decrease angiogenesis, tumor cell survival, and metastasis. Here also therapy resistances occur in view of VEGF / VEGFR inhibitors.

At the same time, the spectrum of side effects of therapy has expanded considerably. Overall, most antineoplastic substances belong to the drugs with the smallest therapeutic window.

Thus, there exists a need in providing a pharmaceutical composition effectively treating and/or preventing a tumor disease, wherein the therapy is safe, preferably provides a broader therapeutic window, results in less side effects and/or overcomes a therapeutic resistance to the therapy regime.

### SUMMARY OF INVENTION

The aforementioned need is attended to at least in part by means of the claimed inventive subject matter. Advantages (preferred embodiments) are set out in the detailed description hereinafter and/or the accompanying figures as well as in the dependent claims.

Accordingly, a first aspect of this invention relates to a pharmaceutical composition comprising a plurality of active agents in a therapeutically effective amount and one or more pharmaceutical excipients, characterized in that
a. at least one active agent inhibits VEGF's and/or VEGFR1-3 signaling, wherein the at least one VEGF's and/or VEGFR1-3 inhibitor is cabozantinib,
b. at least one active agent inhibits SDF1alpha and/or CXCR4 signaling, wherein the at least one SDF1 alpha and/or CXCR4 inhibitor is plerixafor,
c. at least one active agent inhibits EGF and/or EGFR in particular EGFR1-3 signaling, wherein the at least one EGF and/or EGFR1-3 inhibitor is afatinib,
d. at least one active agent inhibits COX2 and/or PGE2 and/or EP signaling, wherein the at least one COX2 and/or PGE2 inhibitor is etoricoxib and
e. at least one active agent inhibits HGF and/or HGFR (MET) signaling, wherein the at least one HGF and/or HGFR (MET) inhibitor is cabozantinib.

A second aspect of this invention relates to a pharmaceutical composition according to the first inventive aspect for use in the treatment or prophylaxis of non-small cell lung cancer (NSCLC) without driver mutations.

The inventive aspects of the present invention as disclosed hereinbefore can comprise - in case it is reasonable for a person skilled in the art - any possible combination of the preferred inventive embodiments as set out in the dependent claims or disclosed in the following detailed description including the experimental section.

### BRIEF DESCRIPTION OF DRAWINGS

Further aspects, characteristics and advantages of the invention will ensue from the following description of the embodiments with reference to the accompanying drawings, wherein
- Fig. 1: is a simplified model of the inventive combination therapy regimen overcoming drug resistances in the therapy of tumor diseases.
- FIGS. 2 a) to f): are diagrams displaying tumor volume and bodyweight during therapy regimen in the respective in vivo models LU 7406 (FIG. 2a), LU7414 (Fig. 2b), LU7177 (Fig. 2c), LU7766 (Fig. 2d), LU7860 (Fig. 2e), and LU7414 (Fig. 2f).

### DETAILLED DESCRIPTION OF EMBODIMENTS

As set out in more detail hereinafter, the inventors of the different aspects of this invention combine for the first time anti-tumor drugs from the group of VEGF and/or VEGFR inhibitors and EGF and/or EGFR, in particular EGFR1-3 inhibitors with active agents blocking the chemokine stromal-cell derived factor-1 (SDF-1 alpha, also known as CXCL12) and C-X-C Motif Chemokine Receptor 4 (CXCR4) axis, and active agents inhibiting cyclooxygenase 2 (COX2) and/or prostaglandin E2 (PGE2) and/or E series of prostaglandin receptors (EP), as well as active agents inhibiting hepatocyte growth factor (HGF) and/or hepatocyte growth factor receptor (HGFR) (MET) according to claim 1 in order to suppress a tumor disease already at lower concentrations compared to a respective monotherapy, in particular a tumor that is resistant to anti-tumor drugs from the group of VEGF and/or VEGFR inhibitors and EGF and/or EGFR, in particular EGFR1-3 inhibitors (see executive example below). In addition, the inventive combination regimen showed at the same time an excellent safety profile of the inventive pharmaceutical composition and might lead to less pronounced side effects in patients. Together with its excellent efficacy this inventive combination is deemed to be superior in comparison to a higher dose therapy regimen of a VEGFR inhibitor monotherapy, e.g. using cabozantinib (a multi-kinase inhibitor that targets MET, AXL, and VEGFR2), or a higher dose therapy regimen of a EGFR, in particular EGFR1-3 inhibitor monotherapy, e.g. using afatinib.

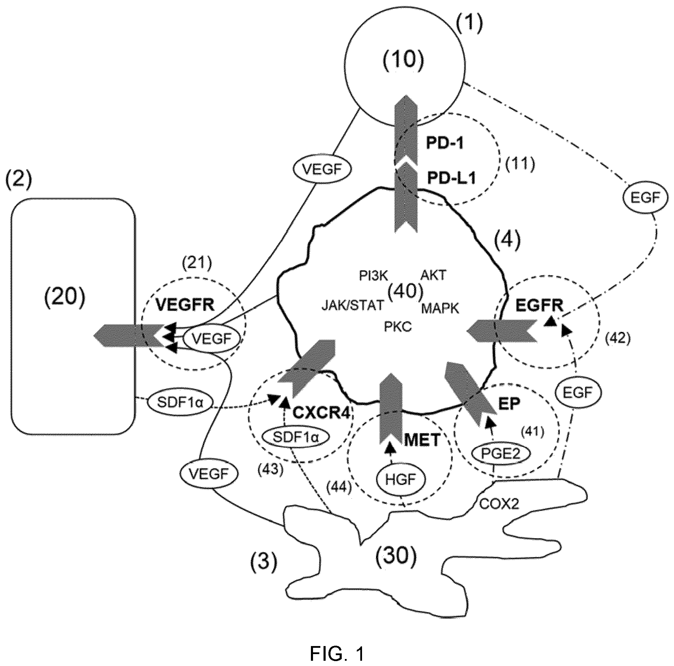

The fact, that the inventive pharmaceutical composition not only simultaneously inhibits VEGF / VEGFR, HGF / HGFR (MET) and EGF / EGFR in particular EGFR1-3 signaling pathways, but in addition simultaneously inhibits SDF-1 / CXCR4, and COX2 / PGE2 / EP signaling pathways seems to be necessary in order to suppress tumor growth by effectively targeting the paracrine signaling from different cell types including immune cells, tumor endothelial cells, cancer associated fibroblasts and tumor cells forming a cellular tumorigenic network (see Fig. 1).

It is surprising, that the inventive combination of active agents overcomes tumor resistances, as it has previously been shown that a combination of cabozantinib and erlotinib, an EGFR inhibitor, did not appear to re-sensitize the resistant patients to erlotinib (Wakelee HA, et al., A phase Iblll study of cabozantinib (XL184) with or without erlotinib in patients with non-small cell lung cancer. Cancer Chemother Pharmacol. 2017 May;79(5):923-932. doi: 10.1007/s00280-017-3283-z.).

Previous studies furthermore showed that plerixafor as an inhibitor of the SDF-1 - CXCR4 axis was found to abolish cabozantinib induced tumor growth inhibition (Patnaik A, et al. Cabozantinib Eradicates Advanced Murine Prostate Cancer by Activating Antitumor Innate Immunity. Cancer Discov. 2017 Jul;7(7):750-765. doi: 10.1158/2159-8290.CD-16-0778. Epub 2017 Mar 8. PMID: 28274958; PMCID: PMC5501767.).

In the context of the present invention the expression *"pharmaceutical composition comprising a plurality of active agents in a therapeutically effective amount"* means that the combination of inhibitory active agents a) to e) and optionally f) are comprised in the pharmaceutical composition in such an amount, that therapeutically suppresses tumor growth, i.e. wherein the volume and/or the weight of tumor or metastatic lesions remain constant or preferably decrease (i.e. as measured in the clinic by the revised RECIST criteria) and/or wherein the events of metastasis are decreased. In view of the synergistic effects provided by the inventive pharmaceutical composition, the concentrations in particular of the respective active agents a), b) and/or c) can be the same or preferably reduced in comparison to the concentration used when applying the respective active agents in monotherapy.

In the context of the present invention the term "active agent" may synonymously be used to "therapeutic agents", "therapeutics", "drugs", "biologicals", "targeted drugs", "immunologicals", "antibodies", "small molecules" and the like.

The first aspect of the present invention thus relates to a pharmaceutical composition comprising a plurality of active agents in a therapeutically effective amount and one or more pharmaceutical excipients, characterized in that
a. at least one active agent inhibits VEGF's and/or VEGFR1-3 signaling, wherein the at least one VEGF's and/or VEGFR1-3 inhibitor is cabozantinib,
b. at least one active agent inhibits SDF1alpha and/or CXCR4 signaling, wherein the at least one SDF1alpha and/or CXCR4 inhibitor is plerixafor,
c. at least one active agent inhibits EGF and/or EGFR in particular EGFR1-3 signaling, wherein the at least one EGF and/or EGFR1-3 inhibitor is afatinib,
d. at least one active agent inhibits COX2 and/or PGE2 and/or EP signaling, wherein the at least one COX2 and/or PGE2 inhibitor is etoricoxib and
e. at least one active agent inhibits HGF and/or HGFR (MET) signaling, wherein the at least one HGF and/or HGFR (MET) inhibitor is cabozantinib.

According to an alternative or additive preferred embodiment of the present invention the inventive composition further comprises
f) at least one active agent inhibiting programmed death 1 receptor (PD-1) and/or programmed death ligand 1 (PD-L1), wherein the PD-1 and/or PD-L1 inhibitor is preferably selected from the group consisting of pembrolizumab, nivolumab, durvalumab, and atezolizumab, preferably durvalumab.

The following table 1 summarizes the information on the selected active agents effectively inhibiting the respective signaling pathways including their presently approved indications including approved dosing as well as a potential dosing in the inventive pharmaceutical combination:

**Tab. 1 Active agents suitable to be used according to the present disclosure within the pharmaceutical composition including approved indications and dosing and potential dosing in a combination regimen, but only the composition as defined in claim 1 is part of the claimes invention. Other compounds listed in Tab. 1 are for information only.**

| **Molecular paracrine signaling pathway** | **Drug** | **Approved indications** (in subgroups thereof) | **Approved dosing** | **Potential dosing in combination regimen** |
|---|---|---|---|---|
| **VEGFA-VEGFR2** | cabozantinib | RCC, HCC | 60mg/day | 20-55mg/day |
| | regorafinib | CRC, HCC, GIST | 160mg/day | 50-150mg/day |
| | bevacizumab | CRC, breast cancer, NSCLC, RCC, ovarian cancer, peritoneal cancer cervical cancer, fallopian tube cancer | 15mg/kg/ 3 weeks | 5-15mg/kg/ 3weeks |
| | ramucirumab | GC, GEJ cancer, CRC, NSCLC | 8mg/kg/2 weeks | 4-7mg/kg/ 2 weeks |
| | pazopanib | RCC, Sarcoma | 800mg/day | 250-750mg/day |
| | sunitinib | GIST, NETs, RCC | 50mg/day | 20-45mg/day |
| | sorafinib | HCC, RCC, DTC, AML | 800mg/day | 250-750mg/day |
| | ponatinib | CML, ALL | 45mg/day | 15-40 mg/day |
| | vandetanib | Thyroid carcinoma | 300mg/day | 100-250mg/day |
| | aixitinib | RCC | 10mg/day | 10mg/day |
| | lenvatinib | RCC, HCC, thyroid carcinoma | 18-24mg/day | 18-24mg/day |
| | ziv-aflibercept | CRC | 4 mg per kg every two weeks | 4 mg per kg every two weeks |
| | tivozanib | Not approved | | |
| | golvatinib | Not approved | | |
| | foretinib | Not approved | | |
| **EGF-EGFRwt** | afatinib | NSCLC | 40mg/day | 10-35mg/day |
| | erlotinib | NSCLC, pancreatic cancer | 150mg/day | 50-145mg/day |
| | cetuximab | CRC, Head & Neck carcinoma | 250mg/m²/ week | 125-240 mg/m²/week |
| | neratinib | Breast cancer | 240mg/day | 150-230mg/day |
| | necitumumab | NSCLC | 800mg/day 1 and 8 in 3 week cycles | 300-750mg/day 1 and 8 in 3 week cycles |
| | gefitinib | NSCLC | 250mg/day | 75-245mg/day |
| | lapatinib | Breast cancer | 1250mg/da y | 500-1200 mg/day |
| | osimertinib | NSCLC | 80mg/day | 20-75mg/day |
| | canertinib | Not approved | | |
| | saracatinib | Not approved | | |
| **SDF1a-CXCR4** | plerixafor | Stem cell mobilization prior transplantation | 20mg/day/5 days | 5-15 mg/day/5days |
| | motixafortide | Not approved | | |
| | LY2510924 | Not approved | | |
| | WZ811 | Not approved | | |
| **COX2-PGE2** | etoricoxib | Arthrosis, rheumatoid arthritis, gout, morbus bechterew, tooth pain | 60mg/day | 10-55mg/day |
| | celecoxib | Arthrosis, rheumatoid arthritis, morbus bechterew | 200mg/day | 50-185mg/day |
| | parecoxib | Post-surgery pain | 40mg/day | 20-35mg/day |
| | rofecoxib | Not approved | | |
| | valdecoxib | Not approved | | |
| **HFG-MET** | cabozantinib | RCC, HCC | 60mg/day | 20-55mg/day |
| | crizotinib | NSCLC | 500mg/day | 100-450mg/kg |
| | tivantinib | NSCLC | 450mg/day | 100-445mg/kg |
| | capmatinib | Not approved | | |
| | tepotinib | Not approved | | |
| | savolitinib | Not approved | | |
| | golvatinib | Not approved | | |
| | foretinib | Not approved | | |

Generally, the inventive composition may contain one, two or more separate pharmaceutical dosage formulations, wherein each dosage formulation contains at least one active agent. The respective pharmaceutical dosage forms may comprise one or more dosage units. In case the pharmaceutical composition includes two or more separate pharmaceutical dosage formulations, the combination of dosage formulations may be commercialized in one kit / package comprising the inventive pharmaceutical composition including the different dosage formulations. As an example, in case one dosage formulation may represent a solid composition, such as a tablet, and one dosage formulation may represent a liquid composition, such as an injection solution, the respective inventive pharmaceutical composition may comprise in the commercialized kit / package one or more units (single tablets) of the solid dosage formulation and one or more units (vials comprising the injection solution) of the liquid dosage formulation. Accordingly, the one, two or more pharmaceutical dosage formulations may independently of each other be the same or different, and may be selected from the group consisting of
i. solid dosage forms for oral or enteral application, wherein solid dosage forms are preferably include tablets, capsules, spheroids, mini-tablets, pellets, granules, and pills;
ii. semi-solid dosage forms for topical application to skin or mucous membranes, wherein semi-solid dosage forms are preferably include creams, gels, ointments, and suppositories; and
iii. liquid dosage forms for oral, external, or parenteral application, wherein liquid dosage forms preferably include mixtures, linctuses, elixirs, syrups, drops, lotions, liniments, collodions, nasal drops, nasal sprays, eye drops, inhalations, aerosols, and injections, e.g., *sub cutan* (s.c.) injections, *intra muscular* (i.m.) injections, *intra venous* (i.v.) injections, *intra peritoneal* (i.p.), *intra pleural* (i.pl.), *intra tumoral* (i.t.), *intra thecal* (i.th.), *intra cerebral*/*lintra cranial* (i.c.) injections.

The one or more pharmaceutical excipients for the respective pharmaceutical formulations may readily be selected as necessary from suitable excipients classes including fillers, diluents, binders, disintegrants, antiadherent, lubricants, glidants, coatings, sorbents, preservatives, antioxidants, flavoring agents, sweeting agents, coloring agents, solvent & co-solvent, buffering agents, chelating agents, viscosity imparting agents, surface active agents, humectants and the like. The technical content regarding the aforementioned solid, semi-solid and liquid dosage forms as well as the aformentioned respective exemplary excipient classes and their respective constitents disclosed in Hand book of pharmaceutical excipients, Edition-Nine, Edited by-Raymond Crowe Paul J Sheskey and Marian E Quinn, Publisher-Pharmaceutical Press; Aulton's Pharmaceutics The design and manufacture of medicines, Edition-Six, Edited by-Michael E.Aulton, Publisher-Elsevier; Ansel's Pharmaceutical Dosage Forms and Drug Delivery Systems, Edition- Twelve, Author Loyd Allen Jr, Timothy B. McPherson,Publisher- Lippincott Williams and Wilkins; Pharmazeutische Technologie: Mit Einführung in Biopharmazie und Biotechnologie, Authors Kurt-Heinz Bauer, Karl-Heinz Frömming and Claus Führer is incorporated herein by reference. As an example, a respectively suitable solid dosage form may be a tablet or a capsule composition and preferably may exhibit in addition to the one or more active agents pharmaceutically acceptable excipients selected from a diluent, a binder, a disintegrant, a lubricant, and optionally a coating. As a further example, a suitable liquid dosage form may be an injection solution, preferably a s.c. injection solution, which may comprise in addition to one or more active agents a suitable solvent, such as water for injections, and a suitable buffer agent / pH adjusting agent.

According to an alternative or additive preferred embodiment of the present invention, the inventive composition may comprise a first pharmaceutical dosage formulation that contains plerixafor, and at least a second pharmaceutical dosage formulation that contains all of cabozantinib, afantinib, eterocoxib and optionally f) at least one active agent inhibiting PD-1 and/or PD-L1 signaling pathways. More preferably, the first pharmaceutical formulation may be a liquid dosage formulation, preferably an s.c. injection dosage form and in particular, and/or wherein the second pharmaceutical dosage form is a solid dosage form, preferably a tablet or capsule and may contain as an example one or more of the remaining active agents. As an example, the first pharmaceutical dosage formulation may be an s.c. injection solution comprising plerixafor in particular in an amount of 24 mg or less per dosage unit, the dosage unit may comprise 1.2 ml. The second pharmaceutical dosage formulation may be selected from a solid dosage formulation, preferably a tablet or a capsule comprising the remaining active agents, i.e. cabozantinib, afatinib, and etoricoxib. In case one or more of the remaining active agents may physically or chemically interact with each other in the respective dosage formulation, e.g. solid dosage formulation, suitable counter measures will be taken as available in the art, such as separating the respective active agents in different compartments of the dosage formulation, masking the respective active agents, etc. and/or separating the respective interacting active agents in separate pharmaceutical dosage formulations.

Regarding a suitable dosing regimen, the active agents of the inventive pharmaceutical composition containing one or more pharmaceutical dosage formulations may generally be administered daily, wherein preferably in this case at least active agents a), b) and/or c) may be administered in a concentration, which is lower than the concentration commonly used in their respective monotherapy in order to decrease side effects. In addition or alternatively, one or more of the active agents of the inventive pharmaceutical composition containing one or more pharmaceutical dosage formulations may be administered less than every day, which may also decrease the risk of side effects.

As shown in the example section, during the complete treatment duration, no tumor outgrowth was observed in either PDX model, indicating a strong therapy efficacy by preventing development of an adaptive resistance against this regimen. After treatment discontinuation growth suppression remained up to 18 days (LU7860) with only slight increase in tumor growth. This indicates a long-lasting and sustained effect of the combination therapy on the tumor biology. In other words, a preferred dose regimen includes the administration of the inventive pharmaceutical composition in intervals from e.g. weeks to months, such as administration of inventive pharmaceutical composition for one or more, preferably five or more days, followed by a one or more, preferably two or more days without administration of the inventive pharmaceutical composition (also called "*drug holiday*")*.* Such interval dose regimen may improve the patient compliance in particular due to a reduced risk of side effects.

According to an alternative or additive preferred embodiment of the present invention, the dose regimen may comprise or consist of
a. the at least one active agent inhibiting VEGF's and/or VEGFR1-3, namely cabozantinib, is administered once daily for one, two, three, four or five days followed by at least one, preferably two days without administration,
b. the at least one active agent inhibiting SDF1alpha and/or CXCR4, namely plerixafor, is administered once daily for one, two, three, four or five days, preferably for one day followed by at least one, preferably five days without administration,
c. the at least one active agent inhibiting EGF and/or EGFR in particular EGFR1-3, namely afatinib, is administered once daily for one, two, three, four or five days followed by at least one, preferably two days without administration,
d. the at least one active agent inhibiting COX2 and/or PGE2 and/or EP, namely etoricoxib, is administered once daily for one, two, three, four or five days followed by at least one, preferably two days without administration,
e. the at least one active agent inhibiting HGF and/or HGFR (MET), namely cabozantinib, is administered once daily for one, two, three, four or five days followed by at least one, preferably two days without administration, and optionally
f. the at least one active agent inhibiting PD-1 and/or PD-L1 that is administered once daily for one, two, three, four or five days followed by at least one, preferably two days without administration.

3. The aforementioned (preferred) embodiments of the first aspect of the present invention may be combined throughout. In particular, the different preferred embodiments of the first aspect of the present invention can alternatively or in addition be combined with each other.
4. All aforementioned embodiments including the combination of preferred embodiments in relation to the first aspect of the present invention can also be used for combination with (preferred) embodiments of the second and third aspect of the present invention.

The second aspect of the present invention relates to a pharmaceutical composition according to the first inventive aspect for use in the treatment or prophylaxis of non-small cell lung cancer (NSCLC) without driver mutations, such as EGFRm, KRAS, FGFR, ALK, cKIT, BRAF.

According to a preferred embodiment the pharmaceutical composition, in particular comprising one or more pharmaceutical formulations is/are respectively provided facilitating enteral administration, preferably oral administration, and/or intranasal administration and/or sub-lingual administration and/or buccal administration. Such an embodiment is preferred, as it may facilitate treatment at home and would not require hospitalization and may improve patient compliance.

As shown in the example section, during the complete treatment duration, no tumor outgrowth was observed in either PDX model, indicating a strong therapy efficacy by preventing development of an adaptive resistance against this regimen. After treatment discontinuation growth suppression remained up to 18 days (LU7860) with only slight increase in tumor growth. This indicates a long-lasting and sustained effect of the combination therapy on the tumor biology. In other words, a preferred dose regimen includes the administration of the inventive pharmaceutical composition in intervals from e.g. weeks to months, such as administration of inventive pharmaceutical composition for one or more, preferably five or more days, followed by a one or more, preferably two or more days without administration of the inventive pharmaceutical composition (also called "*drug holiday*")*.* Such interval dose regimen may improve the patient compliance in particular due to a reduced risk of side effects.

The respective dosing regimens disclosed with respect to the first inventive aspect also apply with respect to the second inventive aspect. In particular, all aforementioned embodiments including a (sub)combination of preferred embodiments disclosed in relation to the first aspect of the present invention can also be used in combination with respect to the second aspect of the present invention.

The present invention is explained further with the aid of the following nonlimiting examples, illustrating the parameters of and compositions employed within the present invention. Unless stated otherwise, all data, in particular percentages, parts and ratios are by weight.

### EXECUTIVE EAMPLES

The following embodiments show the results of treatments of patient-derived NSCLC xenografts (PDX) treated with various anti-cancer monotherapies including chemotherapies, small molecules and monoclonal antibodies and the combination therapy listed within the scope of the present invention (Therapy). Efficacy was measured using the Response Evaluation Criteria in Solid Tumors (RECIST) and according to the measured ratio of the treated tumor (T) versus a placebo control (C) with the largest difference that occurred (optimal).

PD: progressive disease; SD: stable disease; PR: partial response.

### Methods

### Patient-Derived Xenograft (PDX) lung cancer models

The establishment and characterization of PDX mouse models and lung PDX models in particular, was previously described (Tab. 2) (Fichtner at., 2008, Rolff et al., 2016). Generation of the PDX has been approved by the local ethics committee at Charite Berlin and patients have provided informed consent.

In brief, fresh tumor samples were cut into pieces of 3 x 3 mm and were subcutaneously transplanted to Rj:NMRI-Foxn1nu/nu nude recipient mice. Once subcutaneous (s.c.) tumors became palpable, tumor size was measured twice weekly with a digital caliper. Individual tumor volumes (TV) were calculated by the formula V= (length × width²)/2 and related to the values at the first day of treatment (relative tumor volume, RTV). In addition, therapeutic efficacy was assessed by applying Response Evaluation Criteria In Solid Tumors (RECIST) (Eisenhauer et al., 2009). According to these criteria a reduction in tumor volume of at least 30% was defined as partial response (PR), an increase in tumor volume of at least 20% was defined as progressive disease (PD). Stable disease (SD) was defined as neither sufficient reduction to qualify for PR nor sufficient increase to qualify for PD. The Objective Response Rate (ORR) includes all tumors with Complete Response (CR) and PR and the Clinical Benefit Rate includes all tumors with CR, PR and SD expressed in percentage. The body weight (BW) of mice was recorded regularly at least two times per week and the change in body weight was utilized as variable for tolerability.

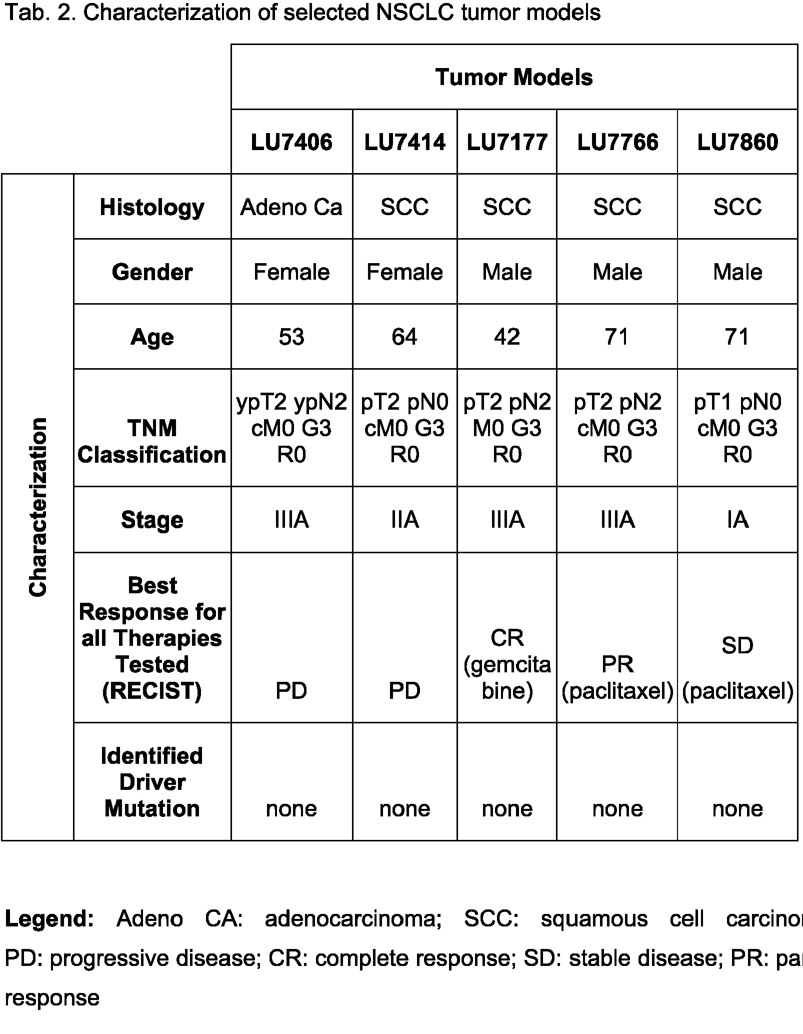

After 25 to 40 days, the mean tumor volume reached the indicated starting volume (150-200 mm3). Mice were randomly assigned to control and treatment groups (1-4 mice per group) and treatment was started on the day of randomization.

The mice were treated using the following drug dosages and treatment schedules: cabozantinib 15 mg/kg, afatinib 15 mg/kg, etoricoxib 10 mg/kg orally and plerixafor 5 mg/kg intra-peritoneally for 5 days on treatment and 2 days off treatment for four cycles in total (Tab. 3a). Common dosing regimes in the respective model for monotherapeutic applications require the once daily application throughout of all active ingredients optionally in a higher dose, such as of cabozantinib 30-60 mg/kg (Paratala et al., 2018; Scott et al., 2018), afatinib 20 mg/kg (Floc'h N et al., 2018), etoricoxib 10 mg/kg (Floc'h N et al., 2018) orally and plerixafor 5 mg/kg intra-peritoneally (Singla et al., 2015). Placebo control mice were treated with corresponding vehicles only (p.o. with 0.5% methyl cellulose and s.c. with aqua ad iniectabilia) or with respective monotherapy at the same dosages. At the end of the experiments, tumors were excised and one half snap frozen and stored at -80 °C for further analyses. The second half was fixed in formalin fixed and embedded in paraffin (FFPE).

All mice were handled in accordance with the Guidelines for the Welfare and Use of Animals in Cancer Research (Workman et al., 2010) and according to the German Animal Protection Law, approved by the responsible local authorities.

**Tab. 3a. Characteristics of the Low Dose Combination Drug Regimen**

| **Target** | **Drug*** | **Route of Administration** | **Dosing in Combination Therapy (****Fig. 2a****-e) and in Monotherapy Controls (****Fig. 2f****)** | |
|---|---|---|---|---|
| | | | **Dosing** | **Schedule** |
| **VEGFR2 (KDR) / MET (HGFR)** | **Cabozantinib** | oral | 15mg/kg | 5 days on / 2 days off for 4 cycles |
| **CXCR4** | **Plerixafor** | intra-peritoneal | 5mg/kg | |
| **EGFRwt** | **Afatinib** | oral | 15mg/kg | |
| **COX2 (PTGS2)** | **Etoricoxib** | oral | 10mg/kg | |

| | | | | |
|---|---|---|---|---|
| * In combination therapy cabozantinib, afatinib and etoricoxib were administered in a single combined gavage dose and plerixafor was administered separately intra-peritoneal | | | | |

**Table 3b. Characteristics of the Drug Regimen of Monotherapy according to state of the art**

| **Target** | **Drug*** | **Route of Adminis-tration** | **Common Dosing *in vivo* Models (state of the art)** | |
|---|---|---|---|---|
| | | | **Dosing** | **Schedule** |
| **VEGFR2 (KDR) / MET (HGFR)** | **Cabozantinib** | oral | 30-60mg/kg (Paratala et al., 2018; Scott et al., 2018) | once daily |
| **CXCR4** | **Plerixafor** | intra-peritoneal | 5mg/kg (Singla et al., 2015) | |
| **EGFRwt** | **Afatinib** | oral | 20mg/kg (Floc'h Net al., 2018) | |
| **COX2 (PTGS2)** | **Etoricoxib** | oral | 10mg/kg (Jayaraman et al., 2010) | |

### Drawings

**Fig. 1** displays a simplified model of the inventive combination therapy regimen overcoming drug resistances by simultaneous targeting interdependent signaling in a cellular tumorigenic network of NSCLC tumors.

In Fig. 1 the following reference signs have the meaning:
(1) Immune cell;
(2) Tumor endothelial cell;
(3) Cancer associated fibroblast;
(4) Tumor cell;
(10) Evading immune destruction;
(20) angiogenesis, proliferation, migration, cell survival and vascular permeability;
(30) secretion of protumorigenic factors;
(40) proliferation, cell survival, metastasis and secretion of VEGF;
(11) active agents inihibiting PD-1-PD-L1 signalling
(21) active agents inihibiting VEGF-VEGFR signalling
(41) active agents inihibiting COX2-PGE2-EP signalling
(42) active agents inihibiting EGF-EGFR signalling
(43) active agents inihibiting SDF1alpha-CXCR4 signalling
(44) active agents inihibiting HGF-MET signalling
PGE2 prostaglandin E2
VEGFR vascular endothelial growth factor receptor;
EGFR epidermal growth factor receptor;
COX2 (PTGS2) cyclooxygenase 2
HGF hepatocyte growth factor.

Signaling axes of PD-1-PD-L1, VEGF-VEGFR, COX2-PGE2-EP, EGF-EGFR, SDF-1-CXCR4, and HGF-MET are exemplary shown for some of the known paracrine pathways binding the cellular tumorigenic network in NSCLC tumors using respective inhibiting active agents (11), (21), (41), (42), (43), and (44), which are inventively selected as follows:
(11) nivolumab, pembrolizumab, stezolizumab, durvalumab;
(21) cabozantinib, regorafinib, bevacizumab, ramucirumab, pazopanib, sunitinib, sorafinib, ponatinib, vandetanib, axitinib, lenvatinib, ziv-aflibercept, tivozanib, golvatinib, foretinib
(41) atoricoxib, celecoxib, parecoxib, rofecoxib, valdecoxib
(42) afatinib, erlotinib, cetuximab, neratinib, necitumumab, gefitinib, lapatinib, osimertinib, canertinib, saracatinib
(43) plerixafor, motixafortide, LY2510924 and WZ811
(44) cabozantinib, crizotinib, tivantinib, capmatinib, tepotinib, savolitinib, golvatinib, foretinib

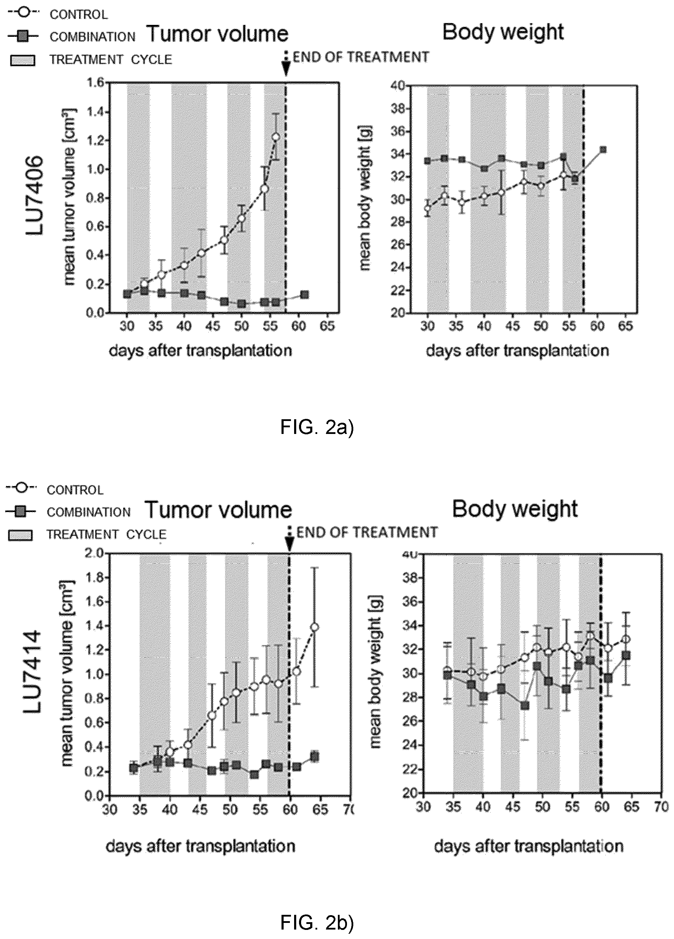

**Figs. 2a) to 2f****)** display *in vivo* efficacy testing of innovative combination treatment regime in selected NSCLC PDX mouse models *versus* monotherapy regiment of the state of the art. In all trials, treatment with the innovative combination was highly effective in preventing tumor propagation and tumor outgrowth achieving stable disease and partial response as best response criteria in all experiments. Stable body weight throughout the complete trials indicate distinguished safety profile of the applied combination treatment.

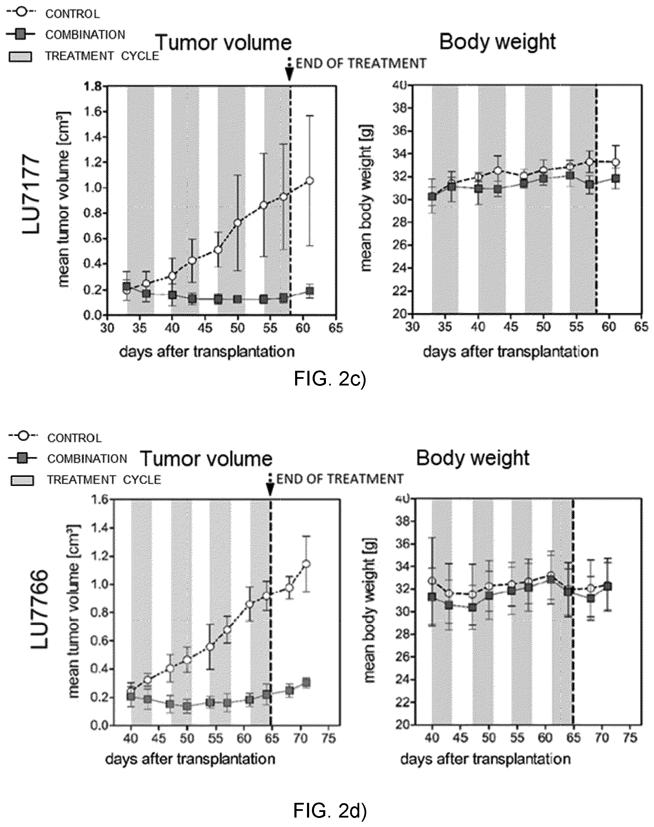

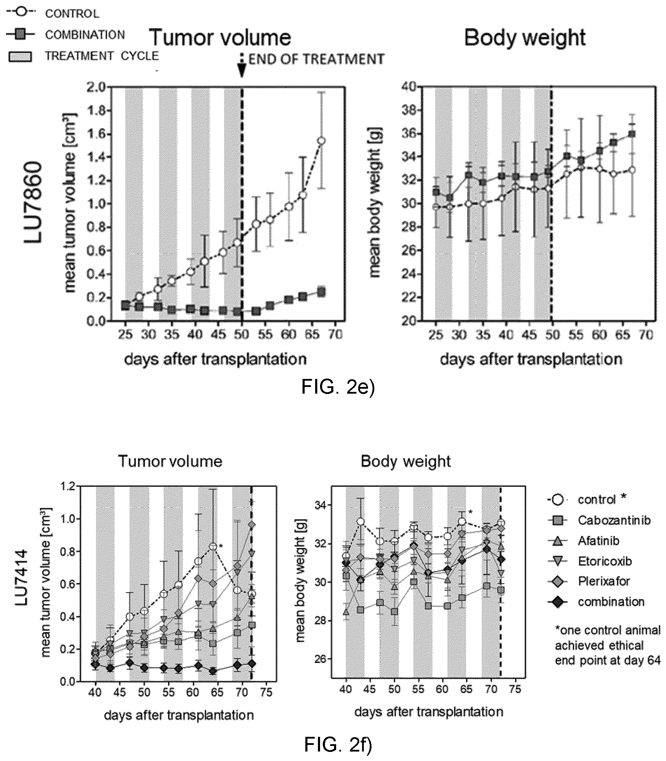

Resistance profiles of all tumor models against carboplatin, paclitaxel, gemcitabine, etoposide, cetuximab, erlotinib and bevacizumab was evaluated in the initial characterization as follows: Fig. 2a. LU7406 and Fig. 2b. LU7414 showed progressive disease (PD) for all tested therapies. Fig. 2c. LU177 showed PD for all but one therapies tested. For gemcitabine complete response (CR) was determined. Fig. 2d. LU7766 showed for four out of the seven tested therapies PD. For carboplatin stable disease (SD), for paclitaxel partial response (PR) and for cetuximab SD was observed. Fig. 2e. LU7860 showed PD for all but one therapies tested. For paclitaxel SD was observed.

In the experiment LU7414 displayed in Fig. 2f. with compounds administered as monotherapies at the same drug dosages used for the innovative combination therapy *versus* control and the innovative combination groups reveal a superior effect of the innovative combination therapy.

### Results

### Complete suppression of tumor growth and absence of toxicity in all NSCLC PDX models treated with the combination therapy regimen

In total, 16 nude mice bearing one of the selected NSCLC PDX tumors were randomized for treatment with the innovative combination therapy regimen consisting of low dose cabozantinib, afatinib, plerixafor and etoricoxib for at least four cycles with 5 days on and 2 days off treatment controlled by a placebo (N=18) *versus* state of the art monotherapy treatment (N=12) group (Tab. 3a). The safety profile of this combination regimen was assessed by the continuous measurement of body weight (BW) of treated mice compared to the placebo group as an indicator for toxicity.

Throughout the experiments only in the LU7414 trial a BW reduction between 5-13% was detected for the innovative combination during the second treatment cycle after day 4 resulting in drug holiday from day 5-7 in this cycle. However, the cumulative dose of these mice was the same compared to all other animals treated with the combination since the first cycle was tested with 6 days on and 1 day off treatment. In none of the remaining mice at any time point a significant difference in BW between the verum and the placebo or the monotherapy group was observed. In general, all mice included in these experiments either gained weight during the course of treatment or remained at the baseline BW level with minimal variances. None of the mice had to be discontinued from treatment, died or had to be euthanized during the course of treatment. This observation reflects the beneficial safety profile of this inventive combination regimen and is in alignment with the low dose therapy compared to regular state of the art concentrations used of these drugs in in vivo experiments (Tab. 3a).

16 out of 16 NSCLC PDX tumors combined from all models showed a treatment response for the applied innovative combination regimen as evaluated per RECIST analysis (Figs. 2a) to 2f)). In 13 tumors a partial response (PR) and in 3 tumors a stable disease (SD) was induced. In contrast, in 29 out of 30 placebo or monotherapy treated tumors progressive disease (PD) was observed. Only for a single tumor of the cabozantinib monotherapy group a partial response was observed and transformed into progressive disease during the later course of treatment (Fig. 2f). Thus, the ORR is 81% and the CBR is 100% across all NSCLC PDX tumors treated with the combination regimen. In none of the mice treated with the innovative combination regime a tumor outgrowth was observed during the treatment period. In contrast, all tumors in the placebo- or monotherapy group showed a progressive disease at the last day of treatment.

In addition, the growth of all tumors in the innovative combination regimen group remained suppressed in the follow up period for up to 18 days (LU7860) (Fig. 2e).

Overall, these data indicate that innovative combined targeting of the paracrine cellular tumorigenic network elicits strong anti-tumoral effects in NSCLC PDX tumors which are characterized by profound resistances to standard therapies.

### Discussion

Despite some recent progress in the treatment of NSCLC, no impactful advances have been in the state of the art when treating tumors without an actionable driver mutation and without the expression of PD-L1 >50% (Herbst et al., 2018).

For the first time, the present inventors showed that the inventive targeting of paracrine signaling from different cell types of the cellular tumorigenic network breaks the intercellular crosstalk and demonstrates a highly effective treatment of tumors that are resistant to monotherapies of chemotherapeutics and targeted drugs against EGFR1-3 and VEGFR. In all treated NSCLC tumors randomly selected from a panel of 38 NSCLC tumors, growth was completely suppressed by the applied low dose inventive combination regimen. Among them were two highly treatment resistant tumors that did not respond to any tested therapy previously (LU7406 and LU7414). During the complete treatment duration, no tumor outgrowth was observed in either PDX model, indicating a strong therapy efficacy by preventing development of an adaptive resistance against this regimen. After treatment discontinuation growth suppression remained up to 18 days (LU7860) with only slight increase in tumor growth. This indicates a long-lasting and sustained effect of the combination therapy on the tumor biology.

The inventive combination therapy regimen can be considered as a low dose treatment regimen. The two drugs with a more pronounced safety profile in this therapy regimen are cabozantinib and afatinib. Cabozantinib is approved for the treatment of renal cell carcinoma and hepatocellular carcinoma and is efficacious in different tumor models at dosages between 30-60 mg/kg once a day (You et al., 2011; Paratala et al., 2018; Kato et al., 2018). Therefore, the inventive drug combination use in our study only includes 50% or less of typically used cabozantinib concentrations in recently published preclinical studies. Afatinib is approved for the treatment of NSCLC either with activating EGFR mutation or in squamous cell carcinoma under or after progression on platinum-based chemotherapy and is efficacious in tumor models at dosages from 20 mg/kg or higher once a day (Floc'h N et al., 2018). In the inventive combination therapy regimen used in this preclinical study, both drugs were used significantly below these state of the art drug doses, each at 15 mg/kg and in addition to that paused after every 5 days of treatment. Plerixafor, approved for stem cell mobilization, and etoricoxib, approved for pain treatment and degenerative and auto-inflammatory diseases, were used at state of the art concentration levels in the respective *in vivo* models (Singla et al., 2015; Jayaraman et al., 2010). The usage of this inventive combination regimen showed an excellent safety profile of the inventive pharmaceutical composition and might lead to less pronounced side effects in patients. Together with its excellent efficacy this inventive combination would be superior to a higher prescribed medication of single cabozantinib or afatinib.

Treatment with the inventive combination therapy does not seem to change the histopathological composition of tumors when compared to placebo treatment regarding the extent of the tumor and the stroma compartment as well as the number of blood vessels. Since the studies have been performed in nude mice, it remains open whether this treatment regimen impacts the infiltration, composition and effector phenotype of T cells. However, our studies also revealed that the expression of PD-L1, whose therapeutic targeting has essentially improved the prognoses of even metastasized NSCLC patients, was not affected by the inventive combination therapy, offering the opportunity of innovative combination therapies including immune checkpoint inhibitors.

Taken together the inventive pharmaceutical composition including the combination of active agents simultaneously inhibiting pathways forming a cellular tumorigenic network in NSCLC tumors can overcome resistance mechanisms of targeted therapy drugs. All of the active agents used in this inventive combination regimen are approved for different indications, and therefore their clinical profiles such as pharmacokinetics, pharmacodynamics and toxicities are well known. They are readily available as study drugs for usage in a clinical trial. In order to identify NSCLC patient subgroups that may respond to this therapeutic concept, inclusion criteria should require the proof of expression of the respective target gene set. Collected tumor specimens can easily be used for mRNA expression analysis by RNA sequencing or by a customized gene expression array. The analysis of mRNA expression as a predictive biomarker is already in clinical development (Schuler et al., 2019).

## Claims

1. A pharmaceutical composition comprising a plurality of active agents in a therapeutically effective amount and one or more pharmaceutical excipients, **characterized in that**
a. at least one active agent inhibits VEGF's and/or VEGFR1-3 signaling, wherein the at least one VEGF's and/or VEGFR1-3 inhibitor is cabozantinib,
b. at least one active agent inhibits SDF1alpha and/or CXCR4 signaling, wherein the at least one SDF1alpha and/or CXCR4 inhibitor is plerixafor,
c. at least one active agent inhibits EGF and/or EGFR1-3 signaling, wherein the at least one EGF and/or EGFR1-3 inhibitor is afatinib,
d. at least one active agent inhibits COX2 and/or PGE2 and/or EP signaling, wherein the at least one COX2 and/or PGE2 inhibitor is etoricoxib and
e. at least one active agent inhibits HGF and/or HGFR (MET) signaling, wherein the at least one HGF and/or HGFR (MET) inhibitor is cabozantinib.

2. The pharmaceutical composition according to claim 1, wherein the composition further comprises
f) at least one active agent inhibiting PD-1 and/or PD-L1, wherein the PD-1 and/or PD-L1 inhibitor is preferably selected from the group consisting of pembrolizumab, nivolumab, durvalumab, and atezolizumab, preferably durvalumab.

3. The pharmaceutical composition according to any one of the preceding claims, wherein the pharmaceutical composition contains one, two or more separate pharmaceutical dosage formulations, wherein each dosage formulation contains at least one active agent.

4. The pharmaceutical composition according to claim 3, wherein the one, two or more pharmaceutical dosage formulations are independently of each other the same or different, selected from the group consisting of
i. solid dosage forms for oral or enteral application, wherein solid dosage forms are preferably include tablets, capsules, spheroids, mini-tablets, pellets, granules, and pills;
ii. semi-solid dosage forms for topical application to skin or mucous membranes, wherein semi-solid dosage forms are preferably include creams, gels, ointments, and suppositories; and
iii. liquid dosage forms for oral, external, or parenteral application, wherein liquid dosage forms preferably include mixtures, linctuses, elixirs, syrups, drops, lotions, liniments, collodions, nasal drops, nasal sprays, eye drops, inhalations, aerosols, and injections.

5. The pharmaceutical composition according to claim 3 or 4, wherein a first pharmaceutical dosage formulation contains plerixafor and at least a second pharmaceutical dosage formulation contains cabozantinib, afatinib, etoricoxib, and optionally f) at least one active agent inhibiting PD-1 and/or PD-L1.

6. The pharmaceutical composition according to claim 5, wherein the first pharmaceutical formulation is a liquid dosage form, preferably an injection dosage form, and/or wherein the second pharmaceutical dosage form is a solid dosage form, preferably a tablet or capsule.

7. The pharmaceutical composition according to any of the preceding claims, wherein the dose regimen may comprise or consist of
a. Cabozantinib being suitable to be administered once daily for up to five days followed by at least one, preferably two days without administration,
b. Plerixafor being suitable to be administered once daily for up to five days, preferably for one day followed by at least one, preferably five days without administration,
c. Afatinib being suitable to be administered once daily for up to five days followed by at least one, preferably two days without administration,
d. Etorixocib being suitable to be administered once daily for up to five days followed by at least one, preferably two days without administration,
e. Cabozantinib being suitable to be administered once daily for up to five days followed by at least one, preferably two days without administration, and optionally
f. At least one active agent inhibiting PD-1 and/or PD-L1 that is administered once daily for up to five days followed by at least one, preferably two days without administration.

8. A pharmaceutical composition according to any one of the preceding claims for use in the treatment or prophylaxis of non-small cell lung cancer (NSCLC) without driver mutations.

9. The pharmaceutical composition for use according to claim 8, wherein the pharmaceutical composition is administered by one or more of the administration routes selected from enteral administration, preferably oral administration, and/or intranasal administration and/or sub-lingual administration and/or buccal administration of the inventive pharmaceutical composition that comprises one or more pharmaceutical formulations.

10. The pharmaceutical composition for use according to claim 8 or 9, wherein the pharmaceutical composition is administered in an interval without administration of the pharmaceutical composition, preferably in an interval comprising one or more, preferably five or more days of administration of the pharmaceutical composition followed by one or more days without administration of pharmaceutical composition.

## Patentansprüche

1. Pharmazeutische Zusammensetzung, die eine Vielzahl von Wirkstoffen in einer therapeutisch wirksamen Menge und einen oder mehrere pharmazeutische Hilfsstoffe umfasst, **dadurch gekennzeichnet, dass**
a. mindestens ein Wirkstoff die VEGF- und/oder VEGFR1-3-Signalübertragung hemmt, wobei der mindestens eine VEGF- und/oder VEGFR1-3-Inhibitor Cabozantinib ist,
b. mindestens ein Wirkstoff die SDF1alpha- und/oder CXCR4-Signalübertragung hemmt, wobei der mindestens eine SDF1 alpha-und/oder CXCR4-Inhibitor Plerixafor ist,
c. mindestens ein Wirkstoff die EGF- und/oder EGFR1-3-Signalübertragung hemmt, wobei der mindestens eine EGF- und/oder EGFR1-3-Inhibitor Afatinib ist,
d. mindestens ein Wirkstoff COX2 und/oder PGE2 und/oder EP-Signalübertragung hemmt, wobei der mindestens eine COX2- und/oder PGE2-Inhibitor Etoricoxib ist und
e. mindestens ein Wirkstoff die HGF- und/oder HGFR (MET)-Signalübertragung hemmt, wobei der mindestens eine HGF- und/oder HGFR (MET)-Inhibitor Cabozantinib ist.

2. Pharmazeutische Zusammensetzung nach Anspruch 1, wobei die Zusammensetzung weiterhin umfasst
f) mindestens einen Wirkstoff, der PD-1 und/oder PD-L1 hemmt, wobei der PD-1- und/oder PD-L1-Inhibitor vorzugsweise aus der Gruppe bestehend aus Pembrolizumab, Nivolumab, Durvalumab und Atezolizumab, vorzugsweise Durvalumab, ausgewählt ist.

3. Pharmazeutische Zusammensetzung nach einem der vorhergehenden Ansprüche, wobei die pharmazeutische Zusammensetzung eine, zwei oder mehrere separate pharmazeutische Dosierungsformulierungen enthält, wobei jede Dosierungsformulierung mindestens einen Wirkstoff enthält.

4. Pharmazeutische Zusammensetzung nach Anspruch 3, wobei die eine, zwei oder mehr pharmazeutische Dosierungsformulierungen unabhängig voneinander gleich oder verschieden sind, ausgewählt aus der Gruppe bestehend aus
i. feste Darreichungsformen zur oralen oder enteralen Anwendung, wobei feste Darreichungsformen vorzugsweise Tabletten, Kapseln, Sphäroide, Minitabletten, Pellets, Granulat und Pillen umfassen;
ii. halbfeste Darreichungsformen für die örtliche Anwendung auf der Haut oder den Schleimhäuten, wobei halbfeste Darreichungsformen vorzugsweise Cremes, Gele, Salben und Suppositorien umfassen; und
iii. flüssige Darreichungsformen zur oralen, äußerlichen oder parenteralen Anwendung, wobei zu den flüssigen Darreichungsformen vorzugsweise Mischungen, Tinkturen, Elixiere, Sirupe, Tropfen, Lotionen, Einreibungen, Kollodien, Nasentropfen, Nasensprays, Augentropfen, Inhalationen, Aerosole und Injektionen gehören.

5. Pharmazeutische Zusammensetzung nach Anspruch 3 oder 4, wobei eine erste pharmazeutische Dosierungsformulierung Plerixafor enthält und mindestens eine zweite pharmazeutische Dosierungsformulierung Cabozantinib, Afatinib, Etoricoxib und gegebenenfalls f) mindestens einen Wirkstoff, der PD-1 und/oder PD-L1 hemmt, enthält.

6. Pharmazeutische Zusammensetzung nach Anspruch 5, wobei die erste pharmazeutische Formulierung eine flüssige Darreichungsform, vorzugsweise eine Injektionsdarreichungsform, ist und/oder wobei die zweite pharmazeutische Darreichungsform eine feste Darreichungsform, vorzugsweise eine Tablette oder Kapsel, ist.

7. Pharmazeutische Zusammensetzung nach einem der vorhergehenden Ansprüche, wobei das Dosierungsschema Folgendes umfassen oder daraus bestehen kann
a. Cabozantinib kann einmal täglich für bis zu fünf Tage verabreicht werden, gefolgt von mindestens einem, vorzugsweise zwei Tagen ohne Verabreichung,
b. Plerixafor kann einmal täglich bis zu fünf Tage lang verabreicht werden, vorzugsweise einen Tag lang, gefolgt von mindestens einem, vorzugsweise fünf Tagen ohne Verabreichung,
c. Afatinib kann einmal täglich für bis zu fünf Tage verabreicht werden, gefolgt von mindestens einem, vorzugsweise zwei Tagen ohne Verabreichung,
d. Etorixocib kann einmal täglich für bis zu fünf Tage verabreicht werden, gefolgt von mindestens einem, vorzugsweise zwei Tagen ohne Verabreichung,
e. Cabozantinib kann einmal täglich für bis zu fünf Tage verabreicht werden, gefolgt von mindestens einem, vorzugsweise zwei Tagen ohne Verabreichung, und gegebenenfalls
f. Mindestens ein Wirkstoff, der PD-1 und/oder PD-L1 hemmt, der einmal täglich bis zu fünf Tage lang verabreicht wird, gefolgt von mindestens einem, vorzugsweise zwei Tagen ohne Verabreichung.

8. Pharmazeutische Zusammensetzung nach einem der vorhergehenden Ansprüche zur Verwendung bei der Behandlung oder Prophylaxe von nichtkleinzelligem Lungenkrebs (NSCLC) ohne Treibermutationen.

9. Pharmazeutische Zusammensetzung zur Verwendung gemäß Anspruch 8, wobei die pharmazeutische Zusammensetzung durch einen oder mehrere der Verabreichungswege verabreicht wird, die ausgewählt sind aus enteraler Verabreichung, vorzugsweise oraler Verabreichung, und/oder intranasaler Verabreichung und/oder sublingualer Verabreichung und/oder bukkaler Verabreichung der erfindungsgemäßen pharmazeutischen Zusammensetzung, die eine oder mehrere pharmazeutische Formulierungen umfasst.

10. Pharmazeutische Zusammensetzung zur Verwendung nach Anspruch 8 oder 9, wobei die pharmazeutische Zusammensetzung in einem Intervall ohne Verabreichung der pharmazeutischen Zusammensetzung verabreicht wird, vorzugsweise in einem Intervall, das einen oder mehrere, vorzugsweise fünf oder mehr Tage der Verabreichung der pharmazeutischen Zusammensetzung gefolgt von einem oder mehreren Tagen ohne Verabreichung der pharmazeutischen Zusammensetzung umfasst.

## Revendications

1. Composition pharmaceutique comprenant une pluralité de substances actives en une quantité thérapeutiquement efficace et un ou plusieurs excipients pharmaceutiques, **caractérisée en ce que**
a. au moins une substance active inhibe la transmission du signal de VEGF et/ou de VEGFR1-3, ledit au moins un inhibiteur de VEGF et/ou de VEGFR1-3 étant le cabozantinib,
b. au moins une substance active inhibe la transmission du signal de SDF1alpha et/ou de CXCR4, ledit au moins un inhibiteur de SDF1alpha et/ou de CXCR4 étant le plerixafor,
c. au moins une substance active inhibe la transmission du signal d'EGF et/ou d'EGFR1-3, ledit au moins un inhibiteur d'EGF et/ou d'EGFR1-3 étant l'afatinib,
d. au moins une substance active inhibe la transmission du signal de COX2 et/ou de PGE2 et/ou d'EP, ledit au moins un inhibiteur de COX2 et/ou de PGE2 étant l'étoricoxib, et
e. au moins une substance active inhibe la transmission du signal de HGF et/ou de HGFR (MET), ledit au moins un inhibiteur de HGF et/ou de HGFR (MET) étant le cabozantinib.

2. Composition pharmaceutique selon la revendication 1, la composition comprenant en outre
f) au moins une substance active qui inhibe PD-1 et/ou PD-L1, l'inhibiteur de PD-1 et/ou de PD-L1 étant de préférence choisi dans le groupe constitué de pembrolizumab, nivolumab, durvalumab et d'atezolizumab, de préférence de durvalumab.

3. Composition pharmaceutique selon l'une des revendications précédentes, la composition pharmaceutique contenant une ou deux formulations posologiques pharmaceutiques distinctes ou plus, chaque formulation posologique contenant au moins une substance active.

4. Composition pharmaceutique selon la revendication 3, ladite une ou lesdites deux formulations posologiques pharmaceutiques ou plus étant indépendamment identiques ou différentes, choisies dans le groupe constitué
i. de formes pharmaceutiques solides pour l'administration orale ou entérale, les formes pharmaceutiques solides comprenant de préférence les comprimés, les capsules, les sphéroïdes, les mini-comprimés, les pastilles, les granulés et les pilules ;
ii. de formes pharmaceutiques semi-solides pour une application locale sur la peau ou les muqueuses, les formes pharmaceutiques semi-solides comprenant de préférence les crèmes, les gels, les pommades et les suppositoires ; et
iii. de formes pharmaceutiques liquides à usage oral, externe ou parentéral, les formes pharmaceutiques liquides comprenant de préférence les mélanges, les teintures, les élixirs, les sirops, les gouttes, les lotions, les liniments, les collodions, les gouttes nasales, les sprays nasaux, les gouttes oculaires, les inhalations, les aérosols et les injections.

5. Composition pharmaceutique selon la revendication 3 ou 4, une première formulation posologique pharmaceutique contenant du plerixafor et au moins une deuxième formulation posologique pharmaceutique contenant du cabozantinib, de l'afatinib, de l'étoricoxib et facultativement f) au moins une substance active qui inhibe PD-1 et/ou PD-L1.

6. Composition pharmaceutique selon la revendication 5, la première formulation pharmaceutique étant une forme pharmaceutique liquide, de préférence une forme pharmaceutique injectable, et/ou la deuxième forme pharmaceutique étant une forme pharmaceutique solide, de préférence un comprimé ou une capsule.

7. Composition pharmaceutique selon l'une des revendications précédentes, le schéma posologique pouvant comprendre ou étant constitué comme suit
a. le cabozantinib peut être administré une fois par jour pendant cinq jours au maximum, suivis d'au moins un, de préférence deux, jours sans administration,
b. le plerixafor peut être administré une fois par jour pendant cinq jours au maximum, de préférence pendant un jour, suivi d'au moins un, de préférence cinq jours sans administration,
c. l'afatinib peut être administré une fois par jour pendant cinq jours au maximum, suivis d'au moins un jour, de préférence deux, sans administration,
d. l'étoricoxib peut être administré une fois par jour pendant cinq jours au maximum, suivis d'au moins un jour, de préférence deux, sans administration,
e. le cabozantinib peut être administré une fois par jour pendant cinq jours au maximum, suivis d'au moins un jour, de préférence deux, sans administration, et, le cas échéant
f. au moins une substance active qui inhibe PD-1 et/ou PD-L1, administrée une fois par jour pendant cinq jours au maximum, suivis d'au moins un jour, de préférence deux, sans administration.

8. Composition pharmaceutique selon l'une des revendications précédentes pour l'utilisation dans le traitement ou la prophylaxie du cancer du poumon non à petites cellules (CPNPC) sans mutations addictives.

9. Composition pharmaceutique pour l'utilisation selon la revendication 8, la composition pharmaceutique étant administrée par une ou plusieurs des voies d'administration choisies parmi l'administration entérale, de préférence l'administration orale, et/ou l'administration intranasale et/ou l'administration sublinguale et/ou l'administration buccale de la composition pharmaceutique selon l'invention, qui comprend une ou plusieurs formulations pharmaceutiques.

10. Composition pharmaceutique pour l'utilisation selon la revendication 8 ou 9, la composition pharmaceutique étant administrée dans un intervalle sans administration de la composition pharmaceutique, de préférence dans un intervalle comprenant un ou plusieurs, de préférence cinq jours ou plus d'administration de la composition pharmaceutique, suivis d'un ou de plusieurs jours sans administration de la composition pharmaceutique.
